# EUROPEAN PATENT APPLICATION

(11) **EP 1 360 938 A1**
(43) Date of publication of application: **12.11.2003**
(21) Application number: 03010176.0
(22) Date of filing: 06.05.2003
(51) Int. Cl.: A61B 17/22, A61B 18/14

(54) **System for operating an ablation generator with dual energy source**

(30) Priority: 07.05.2002 US 143100
(71) Applicant: Irvine Biomedical, Inc., Irvine, California 92614 (US)
(72) Inventor: Chen, Peter C., Irvine, CA 92612 (US); Nguyen, Tho Hoang, Huntington Beach, CA 92646 (US); Hata, Cary K., Tustin, CA 92782 (US); de la Rama, Alan, Cerritos, CA 90703 (US)
(74) Representative: Wallinger, Michael, Dr.

(57) **Abstract**

A catheter system has an RF catheter, an ultrasound catheter, and an ablation generator coupled to the RF generator and the ultrasound generator. The ablation generator is capable of generating RF energy and ultrasound energy.

## Description

### BACKGROUND OF THE INVENTION

### 1. Field of the Invention

The present invention relates to a medical device system and its use for endocardiac mapping and ablation. Specifically, this invention relates to an ablation generator capable of delivering either radiofrequency or ultrasound energy to a catheter for the treatment of various forms of cardiac arrhythmias.

### 2. Description of the Prior Art

Symptoms of abnormal heart rhythms are generally referred to as cardiac arrhythmias, while an abnormally rapid rhythm is referred to as a tachycardia. The presence of an arrhythmogenic region or an accessory pathway in the atria can bypass or short circuit the normal pathway, potentially resulting in very rapid heart contractions, referred to herein as atrial flutter. Atrial flutter is generally characterized by a saw tooth pattern with negative deflections in inferior leads of the ECG, while the atrial rate is in the range of 240-340 beats per minute. Atrial fibrillation is a more complicated case of multiple atrial flutters, resulting in a chaotic and non-regular arrhythmia. Abnormal rapid heart rhythms can also occur in the ventricle, a condition which is better known as ventricular tachycardia.

Treatment of atrial flutter and atrial fibrillation (AFib) may be accomplished by a variety of approaches, including drugs, surgery, implantable pacemakers/defibrillators, and catheter ablation. While drugs may be the choice of treatment for many patients, they only mask the symptoms and do not cure the underlying causes, and they may also cause side effects. In addition, implantable devices only correct the arrhythmia after it occurs. Surgical and catheter-based treatments, on the other hand, will actually cure the problem, usually by ablating the abnormal arrhythmogenic tissues or the accessory pathways responsible for the tachycardia.

Atrial fibrillation is believed to be the result of an aberrant conduction of electrical signals within the atria, resulting in a condition in which the transmission of electrical activity becomes so disorganized that the atria contracts quiveringly. Once considered a benign disorder, AFib is now widely recognized as the cause of significant morbidity and mortality. The most dangerous outcome from AFib is thromboembolism and stroke risk, the latter due to the chaotic contractions of the atria, causing blood to pool. This in turn can lead to clot formation and the potential for an embolic stroke. According to data from the American Heart Association, approximately 75,000 strokes per year are AFib-related.

While radiofrequency (RF) catheter ablation, using current catheter design, has produced promising results, the known catheter usually has only one large electrode for ablation purposes. RF energy delivered to a tip electrode results in a rapid reduction in tissue temperature over a few millimeters, and therefore has been satisfactory for treatment of most accessory pathways and for the treatment of atrioventricular nodal reentrant tachycardia.

However, RF energy is not always appropriate. For example, most ventricular arrhythmias associated with coronary ischemic heart disease may require greater tissue penetration for successful ablation. Left-sided RF ablation may result in stroke, and ablating with RF in the Afib originating in the pulmonary veins may cause stenosis. In the AFib patient, because of the simultaneous occurrence of multiple wavelets of re-entry electrical impulses within the atria, it is necessary to stop the multiple re-entry impulses simultaneously or sequentially through the creation of an endocardial linear lesion.

The use of ultrasound (US) energy has been suggested as being potentially promising in creating deeper lesions in thicker cardiac muscles and also in creating circumferential lesions in an orifice region such as the ostium of the pulmonary vein. Ultrasound is a form of vibration energy (more than 18,000 cycles per second) that is propagated as a mechanical wave by the motion of particles within the medium. This causes compression and rarefaction of the particles, and thus a pressure wave is propagated, associated with the mechanical movement of the particles. In an absorbing medium, the ultrasonic energy is continuously absorbed and converted into heat within the medium. If the temperature elevation is sufficiently high and is kept for a specified time, tissue injury may occur. This thermal effect is similar to that obtained by using other heating systems with equal thermal exposure.

Thus, it is the purpose of the present invention to provide an improved catheter-based ablation system that has a dual energy source (RF and US) in a single generator unit for use in the treatment of a variety of arrhythmogenic clinical indications such as supraventricular tachycardias, atrial flutter, atrial fibrillation, and ventricular tachycardia.

### SUMMARY OF THE DISCLOSURE

In order to accomplish the objects of the present invention, the present invention provides a catheter system having an RF catheter, an ultrasound catheter, and an ablation generator coupled to the RF generator and the ultrasound generator. The ablation generator is capable of generating RF energy and ultrasound energy.

### BRIEF DESCRIPTION OF THE DRAWINGS

FIG. 1 is a system diagram illustrating the components of a catheter-based ablation system according to the present invention.
FIG. 2 is a block diagram of the ablation generator of the system of FIG. 1.
FIG. 3 is a flowchart illustrating the sequence of operation for the system of FIG. 1.
FIG. 4 illustrates how the system of FIG. 1 is used to transmit ultrasound energy during ablation.
FIG. 5 illustrates how the system of FIG. 1 is used to transmit RF energy during ablation.

### DETAILED DESCRIPTION OF THE PREFERRED EMBODIMENTS

The following detailed description is of the best presently contemplated modes of carrying out the invention. This description is not to be taken in a limiting sense, but is made merely for the purpose of illustrating general principles of embodiments of the invention. The scope of the invention is best defined by the appended claims.

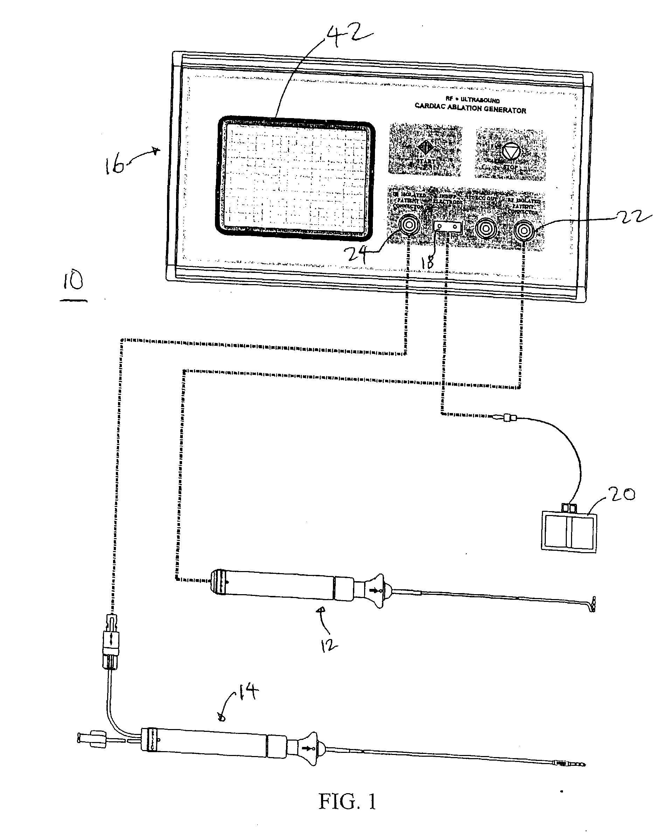

FIG. 1 illustrates the general components of a catheter-based ablation system 10 according to the present invention. The system 10 includes a first catheter device 12 that is adapted to provide RF energy to a treatment site to accomplish ablation. The system 10 also includes a second catheter device 14 that is adapted to provide ultrasound energy to a treatment site to accomplish ablation. The catheter devices 12 and 14 can be provided using catheter constructions that are well known in the art for RF catheter and ultrasound catheters, respectively, and can be operated using RF and ultrasound principles that are well known in the art, so their construction and operation shall not be described in greater detail herein.

The system 10 further includes an ablation generator 16. The ablation generator 16 has an indifferent electrode 18 that is coupled to a patient return pad 20 attached on the back of a patient to complete the RF circuit, and is not needed for use with the ultrasound catheter 14. The ablation generator 16 also includes an RF patient connector 22 that is coupled to the RF catheter 12 to provide RF energy to the RF catheter 12. The ablation generator 16 also includes an ultrasound patient connector 24 that is coupled to the ultrasound catheter 14 to provide ultrasound energy to the ultrasound catheter 14. The ultrasound catheter 14 has an ultrasound transducer enclosed within a balloon that is provided at the distal section of the catheter. A plurality of electrodes is disposed on the distal section of the RF catheter 12, with a conducting wire extending through a lumen of the RF catheter 12 to connect each separate electrode to the connector pins on the proximal end of the catheter handle.

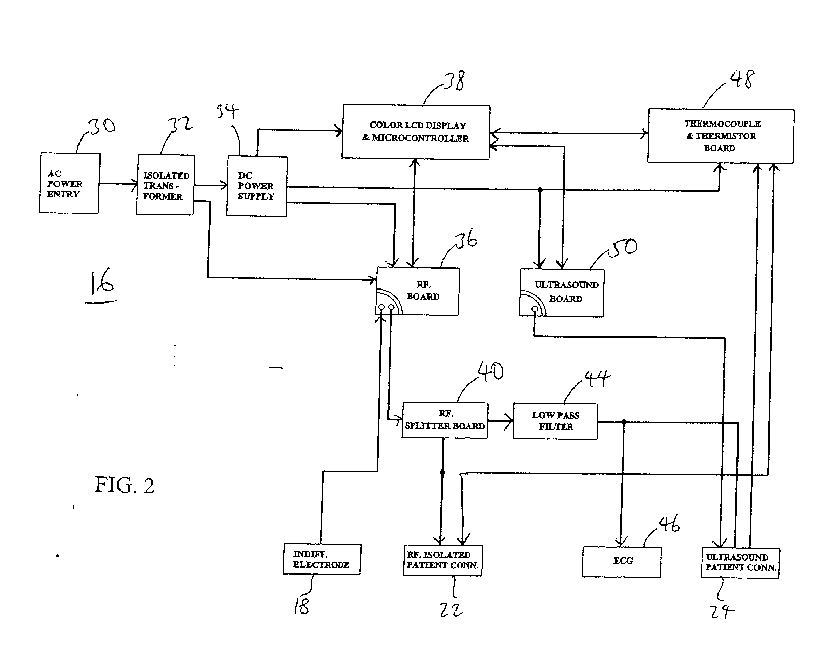

FIG. 2 is a block diagram illustrating the major components of the ablation generator 16. The ablation generator 16 has an AC power entry 30 that receives AC input. The output of the AC power entry 30 is coupled to an input of an isolated transformer 32 which functions to isolate the patient from the electronic circuitry of the system 10. The output of the transformer 32 is coupled to a DC power supply 34 and an RF board 36. The DC power supply 34 converts the AC voltage to DC voltage. The output of the DC power supply 34 is provided to an input of a Color LCD Display and microcontroller 38, an input of an RF board 36, an input of a thermocouple and thermistor board 48, and an input of an ultrasound board 50. The Color LCD Display and microcontroller 38 controls the display of information on a display screen 42 (see FIG. 1), and controls the power, temperature and impedance of the generator 16. Input and output from the Color LCD Display and microcontroller 38, an output from the transformer 32, and one output from the DC power supply 34, are coupled to the RF board 36. The RF board 36 also receives the output of the indifferent electrode 18. The RF board 36 generates a power signal of about 500 kHz and about 150 watts, and provides an output to an input of an RF splitter board 40. The RF splitter board 40 splits the power from the RF board 36 into four channels. The RF splitter board 40 has an output coupled to the RF isolated patient connector 22, and that output is also coupled to an input of a low pass filter 44. Another input to the low pass filter 44 is from the ultrasound patient connector 24.

The output of the low pass filter 44 is connected to an input of an electrocardiogram (ECG) connector 46. An input and an output of the Color LCD Display and microcontroller 38 is provided to the ultrasound board 50, and the output of the ultrasound board 50 is provided to the ultrasound patient connector 24. The ultrasound board 50 generates ultrasound energy of about 7-8 MHz and about 50 watts. A phase-lock loop circuit provided in the ultrasound board 50 functions to maximize the acoustic energy delivered through the ultrasound transducer. A phase detector is provided in the phase-locked loop circuit and functions to detect the impedance of the ultrasound transducer in the ultrasound catheter 14 to enable the generator 16 to provide the appropriate frequency for ultrasound ablation. In particular, the phase detector scans the appropriate current and voltage phase and adjusts to zero phase or the minimum transducer impedance. This minimum impedance allows the ultrasound transducer to vibrate at its natural or resonant frequency, which is the maximum vibrational energy.

In addition, the Color LCD Display and microcontroller 38 is coupled via a two-way connection with the thermocouple and thermistor board 48. The thermocouple and thermistor board 48, which functions as a temperature amplifier, also has inputs that receive the outputs from the DC power supply 34, the RF isolated patient connector 22, and the ultrasound patient connector 24.

Temperature sensors (not shown) are also provided at the proximity of each electrode for the RF catheter 12, and on the balloon or near the ultrasound transducer for the ultrasound catheter 14. The sensors constantly monitor the temperature of the body tissue and relay the temperature to the Color LCD Display and microcontroller 38.

Thus, the DC power supply 34 furnishes the necessary power to both the RF board 36 and the ultrasound board 50. The RF board 36 provides RF energy to the RF catheter 12, while the ultrasound board 50 provides ultrasound energy to the ultrasound catheter 14, all under the control of the Color LCD Display and microcontroller 38. A software program or algorithm can be provided in the Color LCD Display and microcontroller 38 to control the supply of either RF or ultrasound energy. In particular, the microcontroller 38 provides signals to the RF splitter board 40 that are adapted to control the RF energy output to each of the electrodes at a predetermined temperature range. The microcontroller 38 also provides signals to the ultrasound board 50 to control the voltage and current from the microcontroller 38 to maximize the ultrasound energy output to the ultrasound transducer. The sum of the total duration of the RF or ultrasound energy output within the predetermined temperature range that is delivered to each electrode or ultrasound transducer is individually predetermined.

The amount of RF energy density is supplied to each of four electrodes, as well as when selected to provide acoustic energy to the ultrasound transducer on the catheter. The "RF energy density" is defined herein as the RF energy delivered per unit of tissue-contact surface area. In addition, the generator 16 includes a closed-loop control mechanism (provided in the microcontroller 38) for each electrode having a temperature sensor (which can be the same as the temperature sensors mentioned above). To better control the desired lesion characteristics, more RF energy may be applied to an electrode when the measured tissue contact temperature from that electrode is relatively low. On the other hand, RF energy may be minimized when a relatively high tissue contact temperature is detected. The generator 16 can also have a programmed control mechanism for independently selecting and controlling the ablation electrodes of the RF catheter 12 through the RF splitter board 40. In this case, it is possible to select and control the number of electrodes ablated using one of the following modes: a simultaneous mode, a sequential mode, an individual mode, or a combination of the above

For greater control of the lesion characteristics, more ultrasound energy may be delivered to the ultrasound transducer when the measured tissue contact temperature is relatively low. On the other hand, ultrasound energy may be minimized when a relatively high tissue contact temperature is detected.

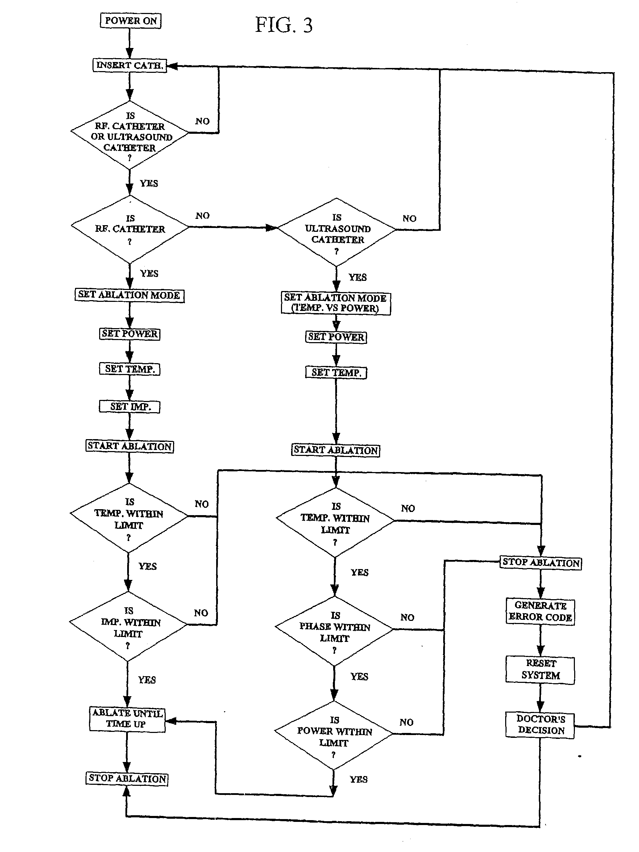

FIG. 3 is a flowchart that illustrates the operation of the ablation generator 16 of FIG. 2. As illustrated in FIG. 3, the generator 16 first determines whether the catheter that is being used is an ultrasound catheter 14 or an RF catheter 12. Once the identity or type of the catheter has been determined, the ablation mode, power and temperature are set, and ablation is then started by providing the selected energy (RF or ultrasound) to the catheter 12 or 14. The generator 16 will also check to see if the temperature, impedance, phase and power (where appropriate) are within pre-selected limits, and the ablation will be stopped if any of these parameters are outside the pre-selected limits. On the other, the ablation is continued as long as these parameters are within the pre-selected limits.

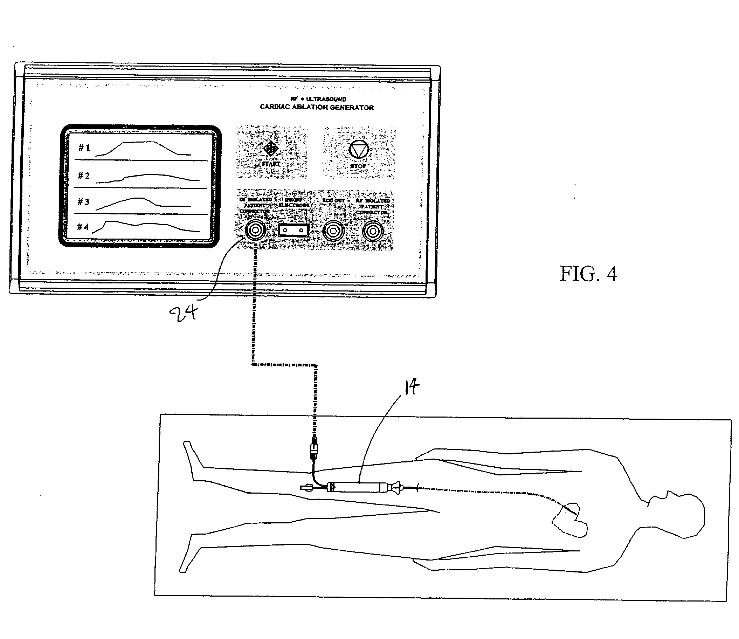

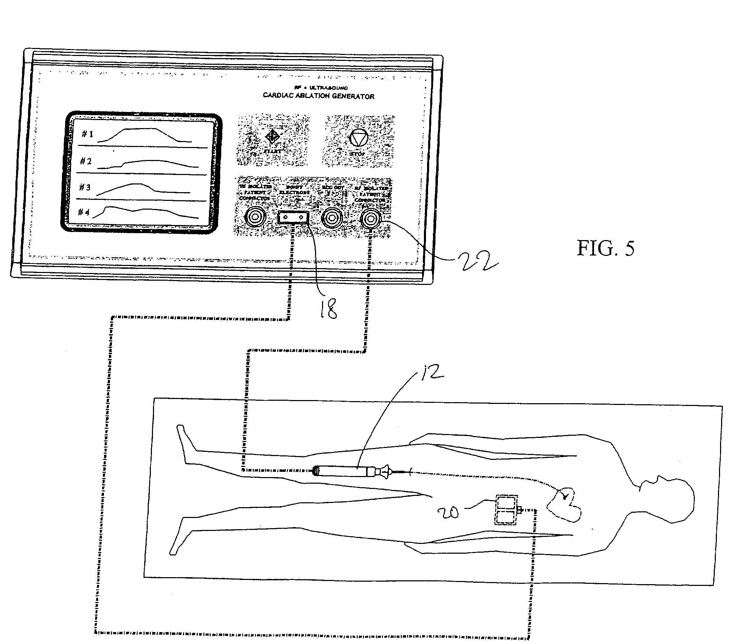

FIG. 4 illustrates how the system of FIG. 1 is used to transmit ultrasound energy during ablation. The ultrasound catheter 14 is coupled to the ultrasound patient connector 24, and the catheter 14 is delivered percutaneously in a minimally-invasive fashion to the treatment area in the patient's heart. FIG. 5 illustrates how the system of FIG. 1 is used to transmit RF energy during ablation. The RF catheter 12 is coupled to the RF patient connector 22, and the catheter 12 is delivered percutaneously in a minimally-invasive fashion to the treatment area in the patient's heart. The patient return pad 20 is coupled to the indifferent electrode 18, and is attached to the back of a patient to complete the RF circuit.

While the description above refers to particular embodiments of the present invention, it will be understood that many modifications may be made without departing from the spirit thereof. The accompanying claims are intended to cover such modifications as would fall within the true scope and spirit of the present invention.

## Claims

1. A catheter system, comprising:
an RF catheter;
an ultrasound catheter; and
an ablation generator coupled to the RF generator and the ultrasound generator and having means for generating RF energy and ultrasound energy.
